# EUROPÄISCHE PATENTSCHRIFT

(11) **EP 0 561 999 B1**
(45) Veröffentlichungstag und Bekanntmachung des Hinweises auf die Patenterteilung: **03.01.1996**
(21) Anmeldenummer: 92902599.7
(22) Anmeldetag: 25.11.1991
(51) Int. Cl.: C07C 305/10, C07C 303/24

(54) **VERFAHREN ZUR HERSTELLUNG VON PARTIALGLYCERIDSULFATEN**
PROCESS FOR THE PRODUCTION OF PARTIAL GLYCERIDE SULPHATES
PROCEDE DE PRODUCTION DE SULFATES DE GLYCERIDES PARTIELS

(30) Priorität: 03.12.1990 DE 4038478
(43) Veröffentlichungstag der Anmeldung: 29.09.1993
(73) Patentinhaber: Henkel Kommanditgesellschaft auf Aktien, D-40191 Düsseldorf (DE)
(72) Erfinder: FABRY, Bernd, D-4052 Korschenbroich (DE); PLOOG, Uwe, D-5657 Haan (DE); BEHLER, Ansgar, D-4250 Bottrop (DE); FEUSTEL, Dieter, D-4019 Monheim (DE)
(86) Internationale Anmeldenummer: EP9102211
(87) Internationale Veröffentlichungsnummer: WO9209570

(56) Entgegenhaltungen:
- EP-A- 0 267 518
- US-A- 2 285 773

## Beschreibung

Die Erfindung betrifft ein Verfahren zur Herstellung von Partialglyceridsulfaten durch Umsetzung von Gemischen bestehend aus Triglyceriden und Glycerin mit gasförmigem Schwefeltrioxid und anchließende Neutralisation der Reaktionsprodukte mit wäßrigen Basen.

Sulfatierte Partialglyceride, insbesondere Monoglyceridsulfate, stellen Aniontenside dar, die sich durch hohes Schaumvermögen, gute Reinigungsleistung und ausgezeichnete dermatologische Verträglichkeit auszeichnen [**Anionic Surfactants, Pt.I, Surfactant science series Vol.7, W.M. Linfield (Ed.), Marcel Dekker Inc., New York, 1976, S. 219**].

Zur Herstellung von Monoglyceridsulfaten geht man gewöhnlich von Glycerin aus, das man zunächst mit Oleum [**US 2,693,479**] oder Chlorsulfonsäure [**JP 78/77014**] zum Glycerinsulfat umsetzt und dann unter Zusatz eines Triglycerids zum Monoglyceridsulfat umestert [**Lipidos 26, 19 (1966)**]. Die deutsche Patentanmeldung **DE-A-38 21 446** offenbart ferner ein Verfahren zur Herstellung von Monoglyceridsulfaten durch Umsetzung von Glycerin mit Chlorsulfonsäure in einem organischem Lösemittel, bei dem zur Umesterung Fettsäuren oder Fettsäureester eingesetzt werden. Die Sulfierung mit Oleum oder Chlorsulfonsäure führt jedoch zu Produkten mit hoher Elektrolytbelastung und ist daher unvorteilhaft.

Aus **Philipp.J.Sci. 311 (1965)** ist bekannt, daß sich Gemische aus Triglyceriden und Glycerin in Gegenwart alkalischer Katalysatoren umestern lassen. In dem genannten Dokument wird ferner vorgeschlagen, die erhaltenen Glycerinfettsäurepartialester mit Schwefelsäure oder Oleum zu sulfatieren.

Die Sulfatierung von Glycerin mit Schwefeltrioxid und die anschließende Umesterung des gebildeten Glycerinsulfats mit Triglyceriden ist aus **US 2,979,521** bekannt. Auf diesen Wegen werden jedoch nur Produkte mit unbefriedigenden Sulfiergraden erhalten.

Desweiteren sind beispielsweise aus der **US 3,634,287** oder der **EP-A-0 267 518** Verfahren zu Herstellung von Partialglyceridsulfaten bekannt, die von Monoglyceriden ausgehen. Partialglyceride wie beispielsweise Monoglyceride stellen jedoch Produkte einer hohen Veredlungsstufe dar, d. h. zu ihrer Herstellung ist ein großer Aufwand an Zeit und Energie erforderlich, so daß sie für die Herstellung von Partialglyceridsulfaten aus ökonomischen Gründen nur eingeschränkt in Betracht kommen.

Die Aufgabe der Erfindung bestand somit darin, ein Verfahren zur Herstellung von Partialglyceridsulfaten zu entwickeln, das frei von den geschilderten Nachteilen ist.

Gegenstand der Erfindung ist ein Verfahren zur Herstellung von Partialglyceridsulfaten durch Sulfierung von Gemischen bestehend aus Triglyceriden und Glycerin, das sich dadurch auszeichnet, daß man
a) Gemische bestehend aus Triglyceriden und Glycerin mit gasförmigem Schwefeltrioxid umsetzt,
b) die sauren Reaktionsprodukte nach der Sulfierung bei erhöhter Temperatur einer Alterung unterwirft und
c) anschließend mit wäßrigen Basen in Gegenwart eines Puffers neutralisiert.

Die Erfindung beruht auf der Erkenntnis, daß durch Alterung der rohen Sulfierungsprodukte und dem Einsatz von Puffern in der der Neutralisation auch dann Partialglyceridsulfate in guten Ausbeuten erhalten werden, wenn man von Rohstoffen einer niedrigen Veredlungsstufe ausgeht.

Bei den Triglyceriden, die im Sinne der Erfindung als Ausgangsstoffe für die Herstellung der Partialglyceridsulfate dienen, handelt es sich um natürliche oder synthetische Vollester des Glycerins mit aliphatischen Carbonsäuren mit 6 bis 22 Kohlenstoffatomen und 0, 1, 2 oder 3 Doppelbindungen. Im einzelnen kommen die Triglyceride der Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Arachinsäure, Gadoleinsäure, Behensäure oder Erucasäure in Betracht. Bevorzugt ist der Einsatz von Glycerintrilaurat.

Wie in der Fettchemie üblich, können die Fettsäurekomponenten der Triglyceride technische Gemische darstellen, wie sie typischerweise in natürlichen Fetten und Ölen, beispielsweise in Kokosöl, Palmöl, Rüböl, Sonnenblumenöl, Korianderöl oder Rindertalg zu finden sind. Bevorzugt ist der Einsatz von gehärtetem oder ungehärtetem Palmkern- oder Kokosöl.

Die Triglyceride werden im Gemisch mit Glycerin in die Sulfierung eingesetzt, wobei Umesterung und Sulfatierung parallel stattfinden. Das molare Verhältnis der Gemische bestehend aus Triglyceriden und Glycerin kann dabei 1 : 1 bis 1 : 4 betragen. Für die gezielte Herstellung von Monoglyceridsulfaten ist es vorteilhaft, ein Verhältnis von 1 : 2 bis 1 : 3 zu wählen.

Die Sulfierung der Gemische bestehend aus Triglyceriden und Glycerin mit gasförmigem Schwefeltrioxid kann in der für Fettsäureniedrigalkylester bekannten Weise [**J.Falbe (ed.), "Surfactants in consumer products", Springer Verlag, Berlin-Heidelberg, 1987, S. 61**] erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Die Sulfierung kann mit einem molaren Einsatzverhältnis von Partialester zu Schwefeltrioxid von 1 : 0,95 bis 1 : 2,2 durchgeführt werden. Im Hinblick auf die Hellfarbigkeit der Produkte hat es sich als optimal erwiesen, ein Einsatzverhältnis von 1 : 0,95 bis 1 : 1,5 zu wählen. Für die Herstellung von Produkten mit hohem Sulfiergrad empfiehlt sich hingegen ein Einsatzverhältnis von 1 : 1,51 bis 1 : 2,2.

Die Sulfierung kann bei Temperaturen von 70 bis 98°C durchgeführt werden. Im Hinblick auf die Herstellung von Produkten mit hohem Sulfiergrad hat es sich als optimal erwiesen, eine Temperatur von 90 bis 95°C zu wählen.

Im Anschluß an die Sulfierung wird das rohe Sulfierprodukt einer Alterung unterworfen. Dieser Schritt kann kontinuierlich, beispielsweise in einer Rohrschlange, oder diskontinuierlich, beispielsweise in einem Kessel durchgeführt werden. Die Alterung kann über einen Zeitraum von 1 bis 240 min, vorzugsweise 5 bis 30 min und bei Temperaturen von 70 bis 98, vorzugsweise 90 bis 95°C durchgeführt werden. Erfolgt die Alterung innerhalb der angegebenen Grenzen bei niedrigen Tenmperaturen, sind zur Erzielung hoher Sulfiergrade lange Verweilzeiten erforderlich und umgekehrt.

Die bei der Sulfierung anfallenden sauren Sulfierprodukte werden nach der Alterung gemeinsam mit wäßrigen Basen in eine wäßrige Pufferlösung eingerührt und neutralisiert, wobei ein pH-Wert von 5,5 bis 9, vorzugsweise 6,5 bis 8 eingehalten werden muß, da andernfalls eine Hydrolyse der Esterbindung oder eine Abspaltung der Sulfatgruppe stattfindet. Als Puffer kommen beispielsweise eine 1 bis 5 gew.-%ige wäßrige Lösungen von Natriumtriphosphat, Natriumhydrogencarbonat oder Citronensäure in Betracht.

Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen gelangen dabei vorzugsweise in Form 5 bis 55 gew.- %iger wäßriger Lösungen zum Einsatz, wobei 5 bis 25 gew.-%ige wäßrige Natriumhydroxidlösungen bevorzugt sind.

Die Sulfierprodukte stellen komplexe Gemische dar, die im wesentlichen Sulfatierungsprodukte der primären sowie sekundären Hydroxylgruppen der durch Umesterung aus den triglyceriden gebildeten Partialglyceride enthalten. Darüberhinaus finden sich offenkettige und cyclische Glycerinsulfate sowie alpha-Glycerinestersulfonate, Seifen, sulfierte Seifen und Glycerin. Geht man von ungesättigten Triglyceriden aus, findet untergeordnet auch noch eine Addition des Schwefeltrioxids an die Doppelbindung der Fettsäurekomponente unter Bildung innenständiger Glycerinestersulfonate statt.

Die Sulfierprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfierprodukte, 0.2 bis 2 Gew.-% Wasserstoffperoxid, berechnet als 100 %ige Substanz, oder entsprechende Mengen Natriumhypochlorit eingesetzt. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

Die Partialglyceridsulfate zeigen oberflächenaktive Eigenschaften und eignen sich zur Herstellung von pulverförmigen oder flüssigen Wasch- und Reinigungsmitteln sowie von Produkten zur Körper- und Haarpflege.

Die folgenden Beispiele sollen den Gegenstand der Erfindung näher erläutern, ohne ihn darauf einzuschränken.

### Beispiele

### Beispiele 1 bis 7:

**Allgemeine Vorschrift zur Sulfierung von Triglycerid/Glycerin-Gemischen.** In einem 1-l-Sulfierreaktor mit Mantelkühlung und Gaseinleitungsrohr wurde eine Mischung aus 673 g (1 mol) gehärtetem Palmkernöl (Verseifungszahl = 250, Iodzahl = 0,9) und 215 g (2,2 mol) Glycerin vorgelegt und mit 80 bis 176 g (1,0 bis 2,2) gasförmigem Schwefeltrioxid umgesetzt. Das SO₃ wurde dazu aus einer entsprechenden Menge 65 gew.-%igen Oleums ausgetrieben, mit Stickstoff auf eine Konzentration von 5 Vol.-% verdünnt und bei einer Temperatur von T = 80 bis 95°C über einen Zeitraum von t = 60 bis 90 min in das Reaktionsgemisch eingeleitet. Das saure Sulfierprodukt wurde anschließend einer Alterung unterworfen und hierzu 30 min bei 90°C gelagert. Danach wurde es zusammen mit 25 gew.-%iger Natriumhydroxidlösung in 200 ml einer 1 gew.-%igen Lösung von Natriumtriphosphat eingerührt und bei pH = 6,5 bis 8 neutralisiert. Die Reaktionsbedingungen und Kenndaten der Produkte sind in Tab.1 zusammengefaßt.

### Beispiel 8:

**Kontinuierliche Sulfierung von Kokosöl und Glycerin.** In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurde ein Gemisch aus 3300 g (5 mol) technischem gehärteten Kokosöl (Verseifungszahl 255, Iodzahl 0,9) und 1012 g (11 mol) Glycerin bei 95°C mit 600 g (5 mol) Schwefeltrioxid umgesetzt. Das Schwefeltrioxid wurde durch Erhitzen aus einer entsprechenden Menge 65 gew.-%igen Oleums ausgetrieben, mit Stickstoff auf eine Konzentration von 5 Vol.-% verdünnt und über eine Düse mit dem Monoglyceridfilm in Kontakt gebracht. Das rohe Sulfierprodukt wurde anschließend einer Alterung unterworfen und hierzu 30 min bei 90°C gelagert. Danach wurde es zusammen mit 25 gew.-%iger Natriumhydroxidlösung in 200 ml einer 1 gew.-%igen Lösung von Natriumtriphosphat eingerührt und bei pH = 6,5 bis 8 neutralisiert. Die Kenndaten des Reaktionsproduktes sind in Tab.1 zusammengefaßt.

### Vergleichsbeispiel 1:

Analog Beispiel 5 wurden Palmkernöl, Glycerin und Schwefeltrioxid bei einer Temperatur von 40°C zur Reaktion gebracht. Die Kenndaten des Produktes sind in Tab.1 zusammengefaßt.

### Vergleichsbeispiel 2:

Analog Beispiel 5 wurden Palmkernöl, Glycerin und Schwefeltrioxid bei einer Temperatur von 95°C zur Reaktion gebracht. Das saure Sulfierprodukt wurde in Abwesenheit des Natriumphosphat-Puffers mit 40 gew.-%iger wäßriger Natriumhydroxid-Lösung neutralisiert. Die Kenndaten des Produktes sind in Tab.1 zusammengefaßt.

Der Gehalt an Glyceridsulfaten (WAS) und die unsulfierten Anteile des Einsatzproduktes (US) wurden nach den DGF-Einheitsmethoden, Stuttgart, 1950-1984, H-III-10 bzw. G-II-6b ermittelt. Der Glycerinsulfatanteil wurde ionenchromatographisch, der Sulfatgehalt potentiometrisch bestimmt und als Natriumsulfat berechnet. Die Bestimmung des Wassergehaltes erfolgte nach der Fischer-Methode. Glycerin wurde enzymatisch, der Seifenanteil dünnschichtchromatographisch bestimmt.

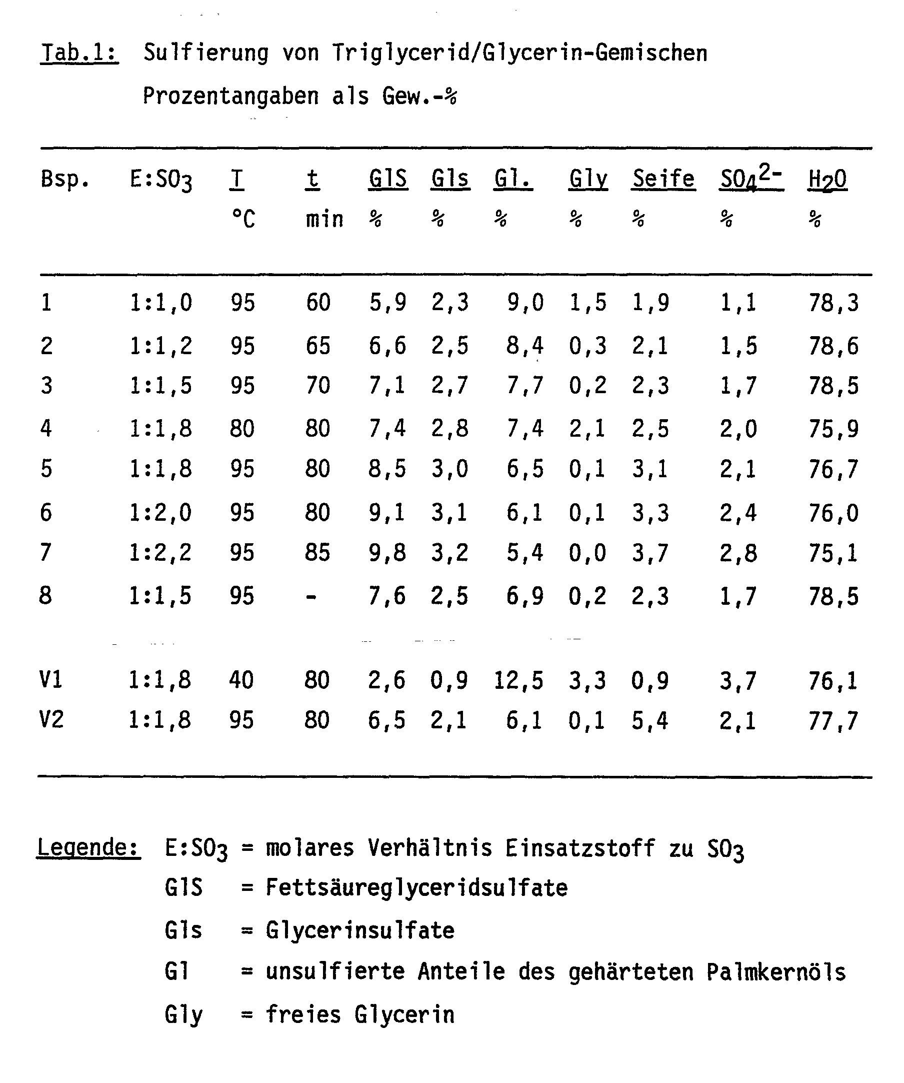

## Patentansprüche

1. Verfahren zur Herstellung von Partialglyceridsulfaten durch Sulfierung von Gemischen bestehend aus Triglyceriden und Glycerin, **dadurch gekennzeichnet,** daß man
a) Gemische bestehend aus Triglyceriden und Glycerin mit gasförmigem Schwefeltrioxid umsetzt,
b) die sauren Reaktionsprodukte nach der Sulfierung bei erhöhter Temperatur einer Alterung unterwirft und
c) anschließemd mit wäßrigen Basen in Gegenwart eines Puffers neutralisiert.

2. Verfahren nach Anspruch 1, **dadurch gekennzeichnet,** daß man die Sulfierung in einem kontinuierlich arbeitenden Reaktor durchführt, der nach dem Fallfilmprinzip arbeitet.

3. Verfahren nach mindestens einem der Ansprüche 1 und 2, **dadurch gekennzeichnet,** daß man Gemische bestehend aus Triglyceriden und Glycerin im molaren Verhältnis von 1 : 1 bis 1 : 4 sulfiert.

4. Verfahren nach mindestens einem der Ansprüche 1 bis 3, **dadurch gekennzeichnet,** daß man die Sulfierung bei einer Temperatur von 70 bis 98°C durchführt.

5. Verfahren nach mindestens einem der Ansprüche 1 bis 4, **dadurch gekennzeichnet,** daß man die Sulfierung mit einem molaren Verhältnis von Triglycerid zu Schwefeltrioxid von 1 : 0,95 bis 1 : 1,5 durchführt.

6. Verfahren nach mindestens einem der Ansprüche 1 bis 4, **dadurch gekennzeichnet,** daß man die Sulfierung mit einem molaren Verhältnis von Triglycerid zu Schwefeltrioxid von 1 : 1,51 bis 1 : 2,2 durchführt.

7. Verfahren nach mindestens einem der Ansprüche 1 bis 6, **dadurch gekennzeichnet,** daß man die Alterung über einen Zeitraum von 1 bis 240 min durchführt.

8. Verfahren nach mindestens einem der Ansprüche 1 bis 7, **dadurch gekennzeichnet,** daß man die Alterung bei einer Temperatur von 70 bis 98°C durchführt.

9. Verfahren nach mindestens einem der Ansprüche 1 bis 8, **dadurch gekennzeichnet,** daß man die Neutralisation mit 5 bis 55 gew.- %igen wäßrigen Basen aus der von Alkalimetallhydroxiden, Erdalkalimetalloxiden und -hydroxiden, Ammoniak, Mono-, Di- und Tri-C₂₋₄-Alkanolaminen sowie primären, sekundären und tertiären C₁₋₄-Alkylaminen gebildeten Gruppe durchführt.

10. Verfahren nach mindestens einem der Ansprüche 1 bis 9, **dadurch gekennzeichnet,** daß man die Neutralisation unter Einhaltung eines pH-Bereiches von 5,5 bis 9 durchführt.

## Claims

1. A process for the production of partial glyceride sulfates by sulfonation of mixtures consisting of triglycerides and glycerol, **characterized in that**
a) mixtures consisting of triglycerides and glycerol are reacted with gaseous sulfur trioxide,
b) the acidic reaction products are subjected to ageing at elevated temperature after sulfonation and
c) are subsequently neutralized with aqueous bases in the presence of a buffer.

2. A process as claimed in claim 1, **characterized in** **that** the sulfonation is carried out in a continuously operated falling-film reactor.

3. A process as claimed in at least one of claims 1 and 2, **characterized in that** mixtures consisting of triglycerides and glycerol are sulfonated in a molar ratio of 1:1 to 1:4.

4. A process as claimed in at least one of claims 1 to 3, **characterized in that** the sulfonation is carried out at a temperature of 70 to 98°C.

5. A process as claimed in at least one of claims 1 to 4, **characterized in that** the sulfonation is carried out with a molar ratio of triglyceride to sulfur trioxide of 1:0.95 to 1:1.5.

6. A process as claimed in at least one of claims 1 to 4, **characterized in that** the sulfonation is carried out with a molar ratio of triglyceride to sulfur trioxide of 1:1.51 to 1:2.2.

7. A process as claimed in at least one of claims 1 to 6, **characterized in that** the ageing step is carried out over a period of 1 to 240 minutes.

8. A process as claimed in at least one of claims 1 to 7, **characterized in that** the ageing step is carried out at a temperature of 70 to 98°C.

9. A process as claimed in at least one of claims 1 to 8, **characterized in that** the neutralization step is carried out with 5 to 55% by weight aqueous bases from the group consisting of alkali metal hydroxides, alkaline earth metal oxides and hydroxides, ammonia, mono-, di- and tri-C₂₋₄-alkanolamines and primary, secondary and tertiary C₁₋₄ alkyl amines.

10. A process as claimed in at least one of claims 1 to 9, **characterized in that** the neutralization is carried out at a pH value of 5.5 to 9.

## Revendications

1. Procédé de production de sulfates de glycérides partiels par sulfonation de mélanges constitués de triglycérides et de glycérine, caractérisé en ce qu'on
a) fait réagir des mélanges de triglycérides et de glycérine avec du trioxyde de soufre gazeux,
b) soumet les produits de réaction acides après la sulfonation à une maturation à température élevée, et
c) neutralise ensuite avec des bases aqueuses en présence d'un tampon.

2. Procédé selon la revendication 1, caractérisé en ce qu'on réalise la sulfonation dans un réacteur fonctionnant en continu et qui travaille selon le principe du ruissellement.

3. Procédé selon au moins l'une des revendications 1 et 2, caractérisé en ce qu'on sulfonate des mélanges constitués de triglycérides et de glycérine en proportion molaire de 1:1 à 1:4.

4. Procédé selon au moins une des revendications 1 à 3, caractérisé en ce qu'on réalise la sulfonation àune température de 70 à 98°C.

5. Procédé selon au moins l'une des revendications 1 à 4, caractérisé en ce qu'on réalise la sulfonation dans une proportion molaire de triglycéride au trioxyde de soufre de 1:0,95 à 1:1,5.

6. Procédé selon au moins l'une des revendications 2 à 4, caractérisé en ce qu'on réalise la sulfonation dans une proportion molaire du triglycéride au trioxyde de soufre de 1:1,51 à 1:2,2.

7. Procédé selon au moins l'une des revendications 1 à 6, caractérisé en ce qu'on réalise la maturation sur un intervalle de temps de 1 à 240 mn.

8. Procédé selon au moins l'une des revendications 1 à 7, caractérisé en ce qu'on réalise la maturation àune température de 70 à 98°C.

9. Procédé selon au moins l'une des revendications 1 à 8, caractérisé en ce qu'on réalise la neutralisation avec des solutions aqueuses de base de 5 à 55 % en poids choisies dans le groupe des hydroxydes de métaux alcalins, des hydroxydes et oxydes de métaux alcalino-terreux, l'ammoniaque, les mono-, di- et tri-(alcanol C₂₋₄) amines ainsi que les (alkyl C₁₋₄) amines.

10. Procédé selon au moins l'une des revendications 1 à 9, caractérisé en ce qu'on réalise la neutralisation en maintenant une zone de pH de 5,5 à 9.
